# EUROPEAN PATENT APPLICATION

(11) **EP 2 194 138 A1**
(43) Date of publication of application: **09.06.2010**
(21) Application number: 08170974.3
(22) Date of filing: 08.12.2008
(51) Int. Cl.: C12P 7/64, C12N 1/12

(54) **Compositions containing docosahexaenoic acid and method for its production**

(71) Applicant: Marenutrica GmbH, 23948 Hohenkirchen (DE)
(72) Inventor: Huusfeldt, Trine, 5450 Otterup (DK)
(74) Representative: Hauck Patent- und Rechtsanwälte

(57) **Abstract**

The present invention relates to a method of producing a fatty acid composition comprising docosahexaenoic acid (DHA) and at least one protein from a heterotrophic microalgae. The invention further relates to the composition obtained by said method and to the use of the composition as a dietary supplement or a pharmaceutical composition.

The method according to the invention utilises three different nutrient media in three different steps, where each nutrient medium is designed for providing optimal conditions for the heterotrophic microalgae in the respective step. Thereby is achieved an improved biomass production compared with the conventional methods, without observing a decrease in the content of the desirable products.

## Description

The present invention relates to a method of producing, from a microalgae, a fatty acid composition comprising docosahexaenoic acid (DHA) and at least one protein. The invention further relates to the composition obtained by said method and to the use of the composition as a dietary supplement or a pharmaceutical composition.

Beneficial effects of increased dietary intake of long chain omega-3 fatty acids in humans, such as docosahexaenoic acid (DHA), are well documented. The effects include the reduction of cardiovascular and inflammatory diseases (i.e. arthritis and atherosclerosis), reduction of depression, increasing length of gestation in the third trimester, and inhibiting tumour growth.

Although a metabolic pathway exists in mammals for the biosynthesis of DHA from dietary linolenic acid, this pathway is bioenergetically unfavourable [Crawford, P. AOCS. Short Course in Polyunsaturated Fatty Acids and Eicosanoids, pp. 270-295 (1987*)*]*.* It is therefore believed that mammals, like fish, obtain most of the DHA from dietary sources, such as algae capable of synthesising DHA.

In this respect several heterotrophic marine microorganisms have been found to produce high levels of the important essential fatty acids, including that of genus *Crypthecodinium* [Jiang and Chen, Process Biochemistry 35 (2000) 1205-1209*;* Jiang and Chen, Journal of Industrial Microbiology & Biotechnology, (1999) Vol. 23, 508-513*;* Vazhappilly and Chen, Journal of the American Oil Chemists Society, (1998) Vol. 75, No. 3 p 393-397]*.* The DHA becomes increasingly concentrated in organisms as it moves up the food chain.

Conventionally humans obtained most of the beneficial omega-3 fatty acids from fish. U.S. Pat. No. 4,670,285 discloses the use of fish oil from fish such as menhaden and herring as a source of C22 omega-3-fatty acids. Indeed, fish oils are the primary commercial source of omega-3-fatty acids. Often, however, fish oils are unusable for human consumption because of contamination with environmental pollutants such as polychlorinated biphenyls (PCB's) or heavy metals.

There are furthermore many problems associated with the recovery of fish oils containing DHA for food uses. However, the use of fish oil as a food additive is limited due to problems associated with its typical fishy smell, unpleasant taste, and poor oxidative stability which makes them unsatisfactory for use in edible products.

Therefore, alternative sources are of interest. Microalgae biomass is particularly suitable for the extraction and purification of individual DHA due to its stable and reliable composition and the use DHA, is encouraged by e.g. the WHO and the American Heart Association.

There have been several attempts in the art to use marine microorganisms for the production of significant quantities of DHA. One such an attempt is disclosed in EP 0 515 460, which describes that it is possible to produce oils containing a high proportion of DHA during cultivation of microorganisms in a fermentor.

However, the DHA obtained by said method has to be recovered by extraction using organic solvents, such as hexane. Such extraction steps are not only expensive and time consuming but also involves potential toxic solvent. As an example can be mentioned that n-hexane in 1994 was included in the list of chemicals on the Toxic Release Inventory [N-Hexane Chemical Backgrounder". National Safety Council. Retrieved on 25 May 2007]. It should furthermore be noted, that in order to obtain the described DHA concentrations, a prolonged culture period is required.

Another problem with the prior art, is that most microorganisms produce two or more polyunsaturated fatty acids (PUFA's) in their lipids. This is often undesirable, as the complexity of the organism's lipid profile will limit the use of the oils in some food and pharmaceutical applications. This is e.g. due to the presence of other undesirable PUFAs in the oil or due to ratios of the different PUFAs falling out of the desirable range for the specific application.

As one example can be mentioned, that eicosapentaenoic acid (EPA) a component of fish oil is an undesirable component in e.g. infant formulas because of its prolonged anticoagulant effects and its depression of arachidonic levels in infants. This has been correlated with reduced rates of infant weight gain (Carlson et al. INFORM 1:306*.*)

Still another problem with known techniques is the use of photosynthetic alga such as *Emiliania* spp. When these species are utilized for the production of DHA, a high yield of DHA may be accomplished but since the algae requires a number of very specific conditions for growth, e.g. light, such processes is complicated, expensive and time consuming, and therefore not suitable for industrial production.

The concept of culturing microorganisms in open or closed systems and harvesting a product from the microorganisms is not new. There are numerous systems used throughout the world in which algae or phytoplankton are grown and harvested, either for direct usage (as for animal food) or for indirect usage (as sources of chemicals such as carotenes).

However, numerous factors are critical in cultivation of very condition-sensitive marine microorganisms, such a microalgae. One of the largest problems in cultivation of microalgae is the fact that very high density cultivation (greater than about 50 g/L microbial biomass) can lead to a decrease in omega-3 fatty acids productivity.

This may be due in part to several factors including the difficulty of maintaining high dissolved oxygen levels due to the high oxygen demand developed by the high concentration of microorganisms in the media. Conventional methods to maintain higher dissolved oxygen level include increasing the aeration rate and/or using pure oxygen instead of air for aeration and/or increasing the agitation rate in the fermentor.

These solutions generally increase the cost of compound production and capital cost of fermentation equipment but can also cause additional problems. For example, increased aeration can easily lead to severe foaming problems in the fermentor, since high cell densities and increased mixing can lead to microbial cell breakage due to increased shear forces in the media. This causes the lipids to be released in media where they can become oxidized and/or degraded by enzymes. Microbial cell breakage is an increased problem in cells that have undergone nitrogen limitation or depletion to induce lipid formation, resulting in weaker cell walls.

Consequently, a system in which precise control of these operating conditions is accomplished will permit not only large scale culturing of algae which cannot now be grown optimally by existing commercial technology but also the mass production of compositions comprising omega-3 fatty acids which are not currently available on an economic basis in the marketplace.

It is therefore a first aspect of the present invention to provide a method for cultivating a heterotrophic microalgae where the optimal conditions are provided throughout the growth of the algea and during production of the desired composition.

It is a second aspect according to the present invention to provide a method for providing a composition comprising DHA and at least one protein where the need for recovering the desired composition from the biomass by extraction using one or more organic solvents is eliminated.

It is a third aspect according to the present invention to provide a method for providing an improve heterotrophic microalgae biomass production.

It is a fourth aspect of the present invention to provide a fatty acid composition comprising both a high concentration of DHA and high concentration of at least one protein, wherein the composition lacks the undesirable fish odour/taste and which has a reasonable shelf life.

It is a fifth aspect of the present invention to provide a fatty acid composition comprising both a high concentration of DHA and high concentration of at least one protein which is free of undesirable contaminants, such a heavy metals and/or organic solvents.

It is a sixth aspect according to the present invention to manufacture said composition over a relatively short period of time and at the same time obtaining a high yield, utilizing an inexpensive medium and simple steps for production.

The new and unique way in which the current invention fulfils one or more of the above-mentioned aspects is to provide a method for producing a fatty acid composition comprising docosahexaenoic acid (DHA) and at least one protein, by cultivating at least one heterotrophic microalgae in the following steps:
a) cultivating the microalgae in a first nutrient medium in order to obtain a first microalgae suspension, said first nutrient medium is arranged for preparing the microalgae for growth,
b) cultivating the first microalgae suspension obtained in a) in a second nutrient medium in order to obtain a second microalgae suspension, said second nutrient medium is arranged for stimulating the growth of the microalgae, and
c) cultivating the second microalgae suspension obtained in b) in a third nutrient medium in order to obtain a third microalgae suspension, wherein the content of nitrogen sources in said third medium is limited in order to induce said microalgae to produce docosahexaenoic acid at a concentration of at least 20 grams per litre of nutrient solution.

In step a) the heterotrophic microalgae adapt themselves to the growth conditions in the first nutrient medium. It is during this period that the individual algae are maturing but not yet are able to divide. During this initial step of the algae growth cycle, synthesis of RNA, enzymes and other molecules occurs.

The medium used in step a) is desigend specifically in order to ensure that the optimal conditions are providing for the algae.

It must be understood that this phase is very important in building new healthy cells that will be able to complete the fermentation. If the conditions in step a) is not optimal each individual cell will not be healthy at the end of step a) and the two following steps will as a result be less efficient and optimal, eventually resulting in reduced concentration of the composition according to the invention.

In step b) the heterotrophic microalgae undergoes a period characterised by cell doubling and rapid growth. The actual rate of this growth depends upon the growth conditions, which affect the frequency of cell division events and the probability of both daughter cells surviving.

The medium used in step b) is desigend specifically in order to ensure that optimal conditions are provided for the algae durig this growth cycle, which preferably is exponential.

In step c) the production of the desired composition is induced by the imposition of a stationary phase of heterotrophic microalgae. This is according to one embodiment of the invention obtaind by using a nutrient medium which is depleted of a nitrogen source. However, this could in addition or alternatively also be achived by lowering or raising the pH-value and/or lowering the temperature of cultivation step c).

By providing different nutrient media in the three different steps, where each nutrient medium provides optimal conditions for the heterotrophic microalgae in the respective step, it is possible to obtain an improved biomass production, (greater than about 100 g/L microbial biomass), compared with the conventional methods, without observing a decrease in the content of the composition according to the invention. At the same time the different steps of the heterotrophic microalgae lifecycle, corresponding to the different steps in the method according to the invention, is completed faster and therefore more economical than in the prior art.

As stated earlier it is important that step a) provides healthy heterotrophic microalgae that will be able to complete fermentation in step b) and c). Conventionally this phase has always been performed in the same medium as the following exponential phase, step b). The inventors have now surprisingly found that when two different nutrient mediums are provides, one for each step, a healtier cell are obtained in step a) which not only has an increased growth rate but said cells are also capable of surviving longer and under harsher conditions; e.g. in respect of increased sear forces in the medium.

The method according to the invention can optionally comprise the additional steps of harvesting the biomass obtained after step a) and/or step b). The harvested biomass obtained can be added to the second and third nutrient medium, respectively. This can have the advantage that any undesirable water-soluble compounds which e.g. can have a negative effect on the growth of the heterotrophic microalgae or the production of the composition according to the invention easily can be removed and thereby reduce the undesirable effect thereof.

During step a) samples can advantagously be obtained from the nutrient medium containg the heterotrophic microalgae in order to determine when the heterotrophic microalgae is ready to be transferred to step b). In this resepct conventional methods of determination cell motality and/or the size of the algae can preferably be used.

Using the specie *Crypthecodinium cohnii* it is desirable to obtain cells having a size of 5-10 µm in step a), since this indicats that the algae have adapted to the first nutrient medium.

Thereafter, the first microalgae suspension is transported or added to a second nutrient medium designed for optimally stimulating the exponential phase. Thereby is it ensured that an desired density of the biomass is obtained faster and more economically. Alternatively the second nutrient medium can be added to the first microalgae suspension.

As for step a) samples can also be obtained during step b) in order to establish when the when the desired biomass of between 50 and 100 g/L nutrient medium is obtained. At this stage *Crypthecodinium cohnii* will preferably have a size of 10-20 µm. When the desired biomass concentration is obtained the second microalgae suspension is transported or added to the third nutrient medium. Alternatively the third nutrient medium can be added to the second microalgae suspension.

During step c) the second microalgae suspension has been introduced to the third nutrient medium thereby inducing production of the composition according to the invention.

The culture is grown for a number of hours which is depending on the used heterotrophic microalgae.

In general, the heterotrophic microalgae are cultivated for a time sufficient to produce the composition according to the invention, usually from about 120 to about 240 hours, although this time is subject to variation.

In any case the culture is grown until the desired concentration of the composition according to the invention is at least 10-weight% of the nutrient medium, preferrably at least 25 weight% of the nutrient medium.

The heterotrophic microalgae used for production is preferably a Dinophyceae. From said class heterotrophic microalgae within the genus of *Crypthecodinium* and *Schizochytrium* are preferred, as these has proven capable of producing relatively large amounts of DHA. However, the specie *Crypthecodinium cohnii* is according to one embodiment of the present invention the most desirable organism to utilize for the production of DHA.

In the marine environment, the heterotrophic microalgae is usually found in full salinity seawater and, as such, is adapted to growth in an environment with a high chloride concentration.

The vast majority of seawater has a salinity of between 3.1% and 3.8%. This means that every 1 kg of seawater has approximately 35 grams of dissolved salts, which consist mostly, but not entirely, of the ions of sodium chlorid: Na⁺, Cl⁻.

Thus, in order to mimic the natural conditions for the heterotrophic microalgae as much as possible, the three nutrient mediums according to the present invention comprises salt at a concentration corresponding to the salinity of seawater, i.e. preferably between about 2% and about 4%, preferably between about 2.1% and about 3.0%, especially about 2.60.

The mediums according to the invention can also comprise other salts naturally occurring in seawater, either alone or in combination with sodium chloride.

The first and second nutrient mediums will contain a nitrogen source, preferably in the form of a yeast extract as this is an inexpensive nitrogen source. The yeast extract is preferably a water-soluble extract of autolyzed yeast cells suitable for use in culture media and could e.g. be a commercially available yeast extract from DIFCO, or MARCOR.

Since the yeast extract is an organic nitrogen source it will also contain a number of other micronutrients. However, a person skilled in the art could easily determine other organic nitrogen sources.

The third nutrient medium further comprises a omega-3 or omega-6 plant oil, such as rapeseed oil and groundnut oil, since the inventors surprisingly have found that the severe foaming problems in the fermentor at high cell densities obtained using the conventional mediums are reduced and in some cases completely eliminated.

When the first nutrient medium comprises a plant oil e.g. rapeseed oil, this has proven especially beneficial for the exponential growth phase of the heterotrophic microalgae in step b) as said algae can obtain a specific growth rate of at least 0.15 h⁻¹ at 27°C and a generation time of at least 3 d⁻¹. As described earlier is microbial cell breakage a problem in cells that have undergone nitrogen limitation or depletion in order to induce lipid formation, when increased shear forces are applied in the media. This is due to the fact that nitrogen limitation or depletion results in weaker cell walls. However, the present inventors have found that the addition of a plant oil, such as rapeseed oil, to the third nutrient medium decrease the microbial cell breakage even when increased shear forces are applied, thereby reducing the amount of lipids in the medium which can become oxidized and/or degraded by enzymes.

Thereby is obtained a composition according to the invention which have fewer undesirable oxidation and/or decomposition products than the oils obtained in the prior art, thereby eliminating the fishy odour and the unpleasant taste normally associated with fish oils.

It has further been discover by the present inventors that the brown stains which conventionally is present on the agitator and the upper half of a fermentor if the nutrient medium contains a yeast extract, are reduced and in some cases even completely eliminated when the nutrient medium comprises a plant oil, such as rapeseed oil.

Conventionally such brown stains are removed using mechanical means, rendering the cleaning both laboriously and expensive. However, using the media according to the present invention eliminates/reduces the need for such mechanical cleaning, whereby e.g. a faster cleaning of the fermentation equipment is obtained.

Those of skill in the art know acceptable carbon sources for use in the second and third nutrient medium. For example, carbon can preferably be provided in the form of glucose. The carbon sources in the first nutrient medium can e.g. be hop and/or malt.

The first nutrient medium can in a preferred embodiment comprise 2-4% NaCl, 0.1-2.0% yeast extract, 0.1-2.0% plant oil (rapeseed oil), 0.1-5.0% ethanol, 10-50% malt, 0.5-3.0% hop, and the remaining being distilled water, as the inventors surprisingly have found that such a nutrient medium ensures that the heterotrophic microalgae are healthier, has a longer lifetime than previously known and is capable of meeting harsher conditions such as increased shear forces in the medium.

The second nutrient medium comprises in a preferred embodiment 2-4% NaCl, 0.5-30% glucose and 0.1-2.0% yeast extract, and the remaining being distilled water. This nutrient medium has the benefits compared to conventional mediums, that the heterotrophic microalgae growths under optimal nutrient conditions, thereby ensuring a faster and more economical production of the desired algae-biomass.

The third nutrient medium, which is designed to initiate a stationary phase of the heterotrophic microalgae, comprises 2-4% NaCl, 0.5-50% glucose, 0.1-2.0% plant oil, such as rapeseed oil, and the remaining being distilled water. As is evident the third nutrient medium is depleted of a nitrogen source. However, this could in addition or alternativly also be achived by lowering or raising the pH-value and/or lowering the temperature of the cultivation process in step c). In general terms it is desirable to stress the heterotrophic microalgae to such an extend that they start producing the desired composition according to the invention.

An alternative way of providing nitrogen deficiencies in the third nutrient medium can be obtained by having a ratio of the carbon source to the nitrogen source, which promotes the efficient production of the composition according to the invention. Using glucose and yeast extract as examples, a preferred ratio of carbon source to nitrogen source is about 10-15 parts glucose to 1 part yeast extract.

The cultivation in step a) and b) can be carried out at any life-sustaining temperature. Generally *C. cohnii* will grow at temperatures ranging from about 15°C to 34°C. Preferably the temperature is maintained at about 25 °C to 30 °C, and most preferably at about 27°C in step a) and b).

Heterotrophic microalgae, which grow at 27°C are preferred, because they will have a faster doubling time, thereby reducing the fermentation time. Appropriate temperature ranges for other microorganisms are readily determined by those of skill in the art.

The production of the composition according to the invention in step c) is preferably achieved at a temperature ranging from about 13°C to about 18°C, with a preferred temperature at about 15 °C, as the inventors have found that this temperature promotes a faster production of said composition.

The cultivation can in all three steps be carried out over a broad pH range, typically from about pH 5.0 to 9.0. Preferably, a pH of about 7.5. A base, such as KOH or NaOH, can be used to adjust the medias pH-value prior to inoculation. During the later stages of the fermentation, the culture medium tends to be lowered, if desired, inorganic pH controls can be used to correct the pH-values during the different steps.

One, more or all of the cultivation steps a), b) and c) are preferably "fed-batch" processes, i.e. it is based on feeding a growth limiting nutrient substrate to the different cultivation steps.

The fed-batch strategy is preferred, as this will provide a high cell density in the fermentor. Mostly the feed solution is highly concentrated to avoid dilution of the bioreactor. The fed-batch process has the advantage that is gives the operator an opportunity of controlling the reaction rate in order to avoid e.g. technological limitations connected to the cooling of the reactor and oxygen transfer. The fed-batch process further allows the metabolic control, to avoid osmotic effects, catabolite repression and production of undesirable side products, such as undesirable PUAF's.

Different strategies can be used to control the growth in a fed-batch process, e.g. nutrient availability, sedimentation rate, temperature, pH, gas exchange rate and cell integrity.

The biomass from the third microalgae suspension obtained in step c) are preferably harvested by conventional means. As examples of suitable harvesting techniques can be mentioned centrifugation, flocculation or filtration, however other techniques are well known for the person skilled in the art.

The harvested biomass can then be cleaned by washing and/or dried, again using conventional techniques and method. The harvested, washed/cleaned and dried biomass has a water content of about 10-weight% to about 50-weight%. The dried microalgae-biomass can in a first embodiment be used directly as a dietary composition according to the invention.

The inventors have proven that the resultant biomass, i.e. the composition according to the invention, comprises at least 25-weight% DHA and at least 10 weight% proteins having a molecular weight between 10 and 250 kDa.

The composition according to the invention will normally contain additional PUFAs in addition to the DHA. These PUFAs can e.g. be one or more of the following: PUFAs : octadecanoic acid (18:0), octadecanoic acid (18:1), linoleic acid (18:2), eicosanoic acid (20:0), and Docosanoic acid (20:0). As used herein, the denotation (18:1) octadecanoic acid means that octadecanoic acid is a carboxylic acid with an 18-carbon chain and one double binding. These denotations are well known in the art and the person skilled in the art would easily understand them.

The content of DHA is advantageously as high as possible in relation to the other PUFAs, and preferably above 25%, more preferably above 45%, especially above 75% and even more especially above 90% of the weight of total PUFAs.

As one example can be mentioned the following fatty acid composition in the composition according to the invention 40% docosahexaenoic acid (22:6), 14% octadecanoic acid (18:0), 22% octadecanoic acid (18:1), 18% eicosanoic acid (20:0), and 6% Docosanoic acid (22:0).

As described above the composition according to the invention comprises at least one protein. Said protein is preferably selected from the proteins naturally synthesised by the heterotrophic microalgae. Due to the relatively high protein content in the composition according to the invention, the composition can also be an alternative protein source or a protein supplement. The protein preferably has a size between about 10 kDa and about 250 kDa, as these proteins have proven especially beneficial and comparable to conventional vegetable proteins.

In the embodiments described above it is contemplated that the entire biomass from step c) is used as the desirable composition according to the invention. Thereby is obtained the advantage over the prior art that additional steps of extracting the desirable compounds are eliminated. Furthermore, since the extraction often involves potential toxic solvents, the final product according to the invention if fee of residues from the organic solvent. A the DHA and other PUFA's are located within the algae, the oils are not subjected to oxidation or degradation, whereby the side-compounds normally associated with the fishy smell and/or taste are not produced. In addition, the composition according to the present invention is cholesterol free, contaminant free [e.g. heavy metals, polychlorobiphenyls (PCBs)], and taste good.

Even though it is not needed to extract the relevant compositions according to the invention, such an extraction step would also be within the scope of the present application. In these cases the composition according to the invention can be extracted from the harvested material using an effective amount of solvent. Those of skill in the art can determine suitable solvents.

Absorption of the composition according to the invention is best achieved in the small intestine of the human or animal body. Thus, the composition according to the invention can in a preferred embodiment be encapsulated with a coating capable of withstanding the effects of the human/animal stomach acid and provide a controlled release in the small intestine. Delivering the composition directly to the small intestine also eliminate any undesirable taste the product may have or is associated with.

Said micro- or nanoencapsulating can according to the present invention be archived by an encapsulation method comprising the following steps:
- preparing core particles of the composition according to the invention
- coating the core particles with a biodegradable release-controlling polymer.

As used herein the term "encapsulation" refers to a range of techniques used to enclose compositions or products in a relatively stable shell known as a capsule, allowing them to, for example, be taken orally or be part of e.g. a gel or cream.

The two main types of capsules are hard-shelled capsules, which are normally used for dry, powdered ingredients, and soft-shelled capsules, primarily used for oils and for active ingredients that are dissolved or suspended in oil. One possible coating technique is the fluidized bed coating technique, which is a simple dipping process.

In the convention fluidized bed process, the fluidized bed is a tank with a porous bottom plate and the polymer is in the form of a powder. The plenum below the porous plate supplies low-pressure air uniformly across the plate. The rising air surrounds and suspends the divided core particles, so the polymer dispersed in the air resembles a boiling liquid. Products that are preheated above the melt temperatures of the powder are dipped in the fluidized bed, where the powder melts and fuses into a continuous coating. A high transfer efficiency results from little drag out and no dripping.

The fluidized bed powder coating method is used to apply heavy coats in one dip, 3 - 10 mils (75 - 250 µm), uniformly to complex shaped products. It is possible to build a film thickness of 100 mils (2500 µm) using higher preheat temperatures and multiple dips.

The person skilled in the art would understand that other conventional coating techniques also can be used according to the invention.

The oil drops are preferrably encapsulated with a polymerisable material of natural origin such as alginat. Using alginat as the encapsulating polymer it has been found that the composition according to the invention is protected against the conditions of the stomach and upper intestine, thereby allowing that the composition according to the invention is introduced into the colon where it may offer it's health benefits.

The microcapsules thereby obtained can either be used as a dietary supplement or as a pharmaceutical composition for reduction of cardiovascular and inflammatory diseases or reduction of depression or increasing length of gestation in the third trimester or and inhibiting tumour growth.

The invention will be explained in greater detail below where further advantageous properties and example embodiments are described with reference to the examples.

### EXAMBLES:

### Example 1

### Preparation of the first, second and third nutrient medium

A preparation of the first, second and third nutrient mediums is prepared as follows:

Each of the following ingredients listed in table 1, except the rapeseed oil, are added one at a time to 500 ml of distilled water with a temperature of 40°C, while it is stirred. One ingredient is completely dissolved before the next ingredient is added.

When all the ingredients are dissolved the pH of the medium is adjusted to 7.6, using 1 N NaOH. The volume is then brought to 1000 ml by the addition of distilled water.

Thereafter the different nutrient media is sterilized by autoclave treatment at 121° C, at 103 kPa above atmospheric pressure, for 15 minutes.

The rapeseed oil is then filter sterilized and separately added.

The media are then cooled at stored for later use.

**Tabel 1**

| **First nutrient medium** | **Second nutrient medium** | **Third nutrient medium** |
|---|---|---|
| 2.6% NaCl | 2.6% NaCl | 2.6% NaCl |
| 1% yeast extract | 20% glucose | 20% glucose |
| 1% rapeseed oil | 1% yeast extract | 1% rapeseed oil |
| 3.5% ethanol | | |
| 30% malt | | |
| 2.5% hop | | |

### Example 2

### Production of the composition according to the invention

### Step a)

Into a 300-liter working volume STF was loaded 100 litre of the first nutrient medium obtained example 1. 10 percent per volume inoculums from a seed fermentor containing about 4 * 10⁶ cells/ml were added to the medium. Agitation was set at 275 rpm, dissolved oxygen (DO) to 4.5 mg/l, the temperature was set to 27°C. The pH-value was continually adjusted to a pH of about 7.5.

Fresh medium (first nutrient medium) was continuously added to the STF, until a complete volume of 300 litre of the first algae suspension was obtained and the algae had a size of 5-10 µm.

### Step b)

Into a 1000-liter working volume STF was loaded 200 litre of the second nutrient medium obtained in example 1. 300 litre of the first algae suspension obtained in step a) was added to said second nutrient medium. Agitation was set at 275 rpm, dissolved oxygen (DO) to 4.5 mg/l, the temperature was set to 27°C. The pH-value was continually adjusted to a pH of about 7.5.

Fresh medium (second nutrient medium) was continuously added to the STF, until a complete volume of 1000 litre of the second algae suspension was obtained.

### Step c)

Into a 25,000-liter working volume STF was loaded 1000 litre of the third nutrient medium obtained example 1. The 1000 litre of the second algae suspension obtained in step b) was added to the medium. Agitation was set at 275 rpm, dissolved oxygen (DO) to 4.5 mg/l, the temperature was set to 15°C. The pH-value was continually adjusted to a pH of about 7.5.

Fresh medium (third nutrient medium) was continuously added to the STF, until a complete volume of 25,000 litre of the third algae suspension was obtained.

The culture was then permitted to grow for an additional time sufficient to ensure that the concentration of DHA was 20 grams per litre of nutrient solution and the concentration of microbial biomass was 100 g/L nutrient medium.

### Harvest

The culture was then harvested by centrifugation with the cell pellet retained. The harvested pellet of cells was frozen and dried (lyophilized) to about 20% moisture content.

The dried pellet could be used directly or be subjected to a micro- or nano-encsapsulation.

## Claims

**1.** A method for producing a fatty acid composition comprising docosahexaenoic acid (DHA) and at least one protein, by cultivating at least one heterotrophic microalgae in the following steps:
a) cultivating the microalgae in a first nutrient medium in order to obtain a first microalgae suspension, said first nutrient medium is arranged for preparing the microalgae for growth,
b) cultivating the first microalgae suspension obtained in a) in a second nutrient medium in order to obtain a second microalgae suspension, said second nutrient medium is arranged for stimulating the growth of the microalgae, and
c) cultivating the second microalgae suspension obtained in b) in a third nutrient medium in order to obtain a third microalgae suspension, wherein the content of nitrogen sources in said third medium is limited in order to induce said microalgae to produce docosahexaenoic acid at a concentration of at least 20 grams per litre of nutrient solution.

**2.** The method according to claim 1, wherein the method further comprises harvesting the algae-biomass from the third microalgae suspension.

**4.** The method according to claim 1 or 2, wherein the method further comprises cleaning and/or purifying and/or drying the harvested algae-biomass.

**5.** The method according to claim 1, 2 or 3, wherein at least one of the cultivation steps a), b) c) is a fed-batch fermentation.

**8.** The method according to any of the preceding claims, wherein the heterotrophic microalgae is of the class Dinophyceae, preferably of the genus *Crypthecodinium* or *Schizochytrium*, preferably the species *Crypthecodinium cohnii*.

**9.** The method according to any of the preceding claims, wherein the temperature in the first cultivation step a) and/or second cultivation step b) are about 25°C to about 30°C, preferably about 27°C.

**10.** The method according to any of the preceding claims, wherein the temperature in the third cultivation step c) are about 13°C to about 18°C, preferably about 15°C.

**11.** The method according to any of the preceding claims, wherein the first, second and third nutrient medium comprises a salt, preferably a naturally occurring salt in seawater such as NaCl and a carbon source preferably in the form of glucose and/or plant-rapeseed oil and wherein the first and second nutrient medium comprises a nitrogen source, preferably in the form of a yeast extract.

**14.** The method according to any of the preceding claims, wherein the first nutrient medium comprises 2-4% NaCl 0.1-2.0% yeast extract, 0.1-2.0% rapeseed oil, 0.1-5.0% ethanol, 10-50% malt, 0.5-3.0% hop, the remaining being distilled water.

**15.** The method according to any of the preceding claims, wherein the second nutrient medium comprises 2-4% salt, 0.5-30% glucose and 0.1-2.0% yeast extract.

**16.** The method according to any of the preceding claims, wherein the third nutrient medium comprises 2-4% salt, 0.5-50% glucose and 0.1-2.0% rapeseed oil.

**17.** The method according to any of the preceding claims, wherein the first, second and third culture medium has a pH between 5.0 and 9.0, preferably, a pH about 7.5.

**18.** A nutrient medium according to any of the claims 14 - 17.

**19.** A composition obtainable by the method according to any of the claims 1 - 17, comprising at least 25 weight% DHA and at least 10 weight% of the at least one protein

**20.** The composition according to claim 19, wherein the at least one protein is selected from the proteins naturally synthesised by the heterotrophic microalgae and wherein said protein has a size between 10 kDa and 250 kDa.

**21.** A method for micro- or nanoencapsulating the composition according to the claims 19 or 20, said method comprises
- preparing core particles of the composition according to any of claims 19 or 20, and
- coating the core particles with a coating polymer.

**22.** The method according to claim 21, wherein the coating polymer is alginate.

**23.** Micro-or nanocapsules obtained by the method according to any of the claims 21 or 22.

**24.** A dietary supplement comprising the micro-or nanocapsules according to claim 23 or the composition according to any of the claims 19 or 20.

**25.** A pharmaceutical composition comprising the micro-or nanocapsules according to claim 23 or the composition according to any of the claims 19 or 20.

**26.** Use of the pharmaceutical composition according to claim 25 for reduction of cardiovascular and inflammatory diseases or reduction of depression or increasing length of gestation in the third trimester or and inhibiting tumour growth.
